Europäisches Patentamt

(19) European Patent Office

Office européen des brevets

(11) Veröffentlichungsnummer: **0 155 454**

**A1**

(12) **EUROPÄISCHE PATENTANMELDUNG**

(21) Anmeldenummer: 85100605.6

(22) Anmeldetag: 22.01.85

(51) Int. Cl.⁴: **A 61 K 31/55**
C 07 D 471/04
//(C07D471/04, 243:00, 221:00)

(30) Priorität: 03.02.84 DE 3403693

(43) Veröffentlichungstag der Anmeldung:
25.09.85 Patentblatt 85/39

(84) Benannte Vertragsstaaten:
AT BE CH DE FR GB IT LI LU NL SE

(71) Anmelder: Dr. Karl Thomae GmbH
Postfach 1755
D-7950 Biberach (Riss)(DE)

(72) Erfinder: Tauber, Oswald, Dr.
Beim Forhäldele 7
D-7950 Biberach 1(DE)

(72) Erfinder: Schmidt, Günther, Dr. Dipl.-Chem.
Johann-Seb.-Bach-Strasse 27
D-7900 Biberach 1(DE)

(54) Verwendung von Pirenzepin, seinen Salzen bzw. Pirenzepin-haltigen Arzneimitteln zur Behandlung des Colon irritabile.

(57) Beschrieben wird die neue Verwendung von Pirenzepin bzw. seinen pharmakologisch verträglichen Salzen zur Behandlung des Colon irritabile. Beschrieben werden auch Arzneimittelformen, die diesem Zweck dienen.

EP 0 155 454 A1

DR. KARL THOMAE GMBH
D-7950 Biberach 1

Case 5/902
Dr.Bu./pf.

**Verwendung von Pirenzepin, seinen Salzen bzw. Pirenzepin-
haltigen Arzneimitteln zur Behandlung des Colon irritabile**

Die Erfindung betrifft die Verwendung von Pirenzepin, seinen
Salzen bzw. Pirenzepin-haltigen Arzneimitteln zur Behandlung
des Colon irritabile.

Pirenzepin (5,11-Dihydro-11-[(4-methyl-piperazin-1-yl)-
acetyl]-6H-pyrido[2,3-b][1,4] benzodiazepin-6-on) und seine
pharmakologisch verträglichen Salze, z.B. Pirenzepin-dihydrochlorid-monohydrat, haben Verwendung als Ulcustherapeutikum gefunden. Es handelt sich um eine antimuskarinisch wirksame Substanz, die eine selektive Hemmung der Säuresekretion
ermöglicht, ohne die unerwünschten atropinartigen Nebenwirkungen der klassischen Anticholinergica zu zeigen, nämlich
die Nebenwirkungen am Magendarmtrakt, am Herzen, an den
Speicheldrüsen und den Augen. Es wurde vor allem keine
Beeinflussung der Magendarmmotorik beobachtet, die ebenfalls
den Anticholinergika zu eigen ist.

Überraschenderweise wurde nun gefunden, daß Pirenzepin, obwohl es bekanntermaßen die Magendarmmotorik nicht beeinflußt, in der Lage ist, auch in der Indikation Colon irritabile therapeutisch wirksam zu werden. Aufgrund bisher bekannter pharmakologischer, klinisch-experimenteller und
klinischer Daten war diese neugefundene Indikation des Pirenzepins nicht erkennbar. Pirenzepin vermindert die Neostigmin-induzierten segmentalen Kolonkontraktionen, die sich bei Colon irritabile als Schmerz bemerkbar machen, ohne die propulsive motorische Aktivität einzuschränken. Hierin liegt aber ein wesentlicher, bisher nicht bekannter und nicht vorhersehbarer Vorteil des Pirenzepins gegenüber den heute geübten Therapieversuchen in der angegebenen Indikation.

Zur Behandlung des Colon irritabile wird Pirenzepin und seine pharmakologisch verträglichen Salze wie z.B. Pirenzepin-dihydrochloridmonohydrat, gegebenenfalls in Gegenwart üblicher Träger- und/oder Hilfsstoffe, oral in einer Dosierung von 10 bis 100 mg, 1- bis 2-mal täglich an Erwachsene verabreicht. Die bevorzugte Einzeldosis beträgt dabei 20 bis 50 mg, die bevorzugte Tagesdosis 50 bis 100 mg. Bei parenteraler Verabreichung liegen bei Erwachsenen die Einzeldosen zwischen 1 und 20 mg, bevorzugt 2 und 10 mg.

In der bisherigen Therapie des Colon irritabile wurden bis heute klassische Anticholinergika und Spasmolytika zum Einsatz gebracht. Der therapeutische Erfolg war oft sehr unbefriedigend, zumal alle bei den Anticholinergika bekannten Nebenwirkungen, wie Obstipation, Mundtrockenheit, Akkomodationsstörungen und Frequenzsteigerungen des Herzens, in Kauf genommen werden mußten.

Das Colon irritabile ist ein Sammelbegriff für Symptome im Verdauungskanal, wobei Leibschmerzen, Durchfall und Verstopfung dominieren. Die Beschwerden des Patienten variieren in Dauer und Intensität. Anatomische Veränderungen im Magendarmtrakt findet man in der Regel nicht. Das Colon irritabile wurde deshalb auch als funktionelle Störung des Magendarmtraktes definiert; da man seine Ursachen nicht kannte, reihte man es in die Gruppe der psychosomatischen Krankheiten ein. Psychische Spannungen und Streß können die Beschwerden auslösen und verstärken und ulkusähnliche Schmerzen vortäuschen. Man kommt wahrscheinlich der Definition des Krankheitsbildes am nächsten, wenn man es als Disharmonie innerhalb des vegetativen Nervensystems bezeichnet, mit anderen Worten als ein Mißverhältnis zwischen sympathischen und parasympathischen Einflüssen im vegetativen Nervensystem.

Die Wirksamkeit auf das Colon irritabile ist bei einer Dosierung von 2 x 50 mg/Tag belegt, während tierexperimentell nachgewiesen ist, daß eine der Atropinwirkung vergleichbare Beeinflussung der Colon-Muskulatur erst bei 120- bis 240-facher Überdosierung, im Vergleich zu der wirksamen Dosierung, beobachtet werden kann. Eine Atropin-ähnliche Wirkung auf das Colon durch Pirenzepin findet also bei therapeutischer Dosierung nicht statt. Obwohl Pirenzepin und Atropin bei der Hemmung der Magensekretion einen vergleichbaren Wirkungsmechanismus aufweisen, unterscheidet sich die Wirkung an der Colon-Muskulatur vollständig. Während Atropin bereits in therapeutisch niedriger Dosierung eine Verminderung der Darmmotilität bewirkt (vgl. Leitold et al., Therapie-Woche 27, 9 (1977), Seiten 3 bis 9), ist ein solcher Effekt auch bei therapeutischer Höchstdosen von Pirenzepin nicht sichtbar. Bei der Anwendung von $H_2$-Rezeptorenblockern ist über eine therapeutisch anwendbare Beeinflussung des Colons bisher nichts berichtet worden.

Bei klinischen Untersuchungen an 30 Patienten mit dem angegebenen Beschwerdebild Colon irritabile, wobei sich trotz umfangreicher diagnostischer Maßnahmen bei keinem der Patienten ein pathologischer Befund ergab, wurde Pirenzepin-dihydrochloridmonohydrat zum Einsatz gebracht. Da es für einen möglichen Therapieerfolg bei der geschilderten Indikation keinen objektivierbaren Parameter gibt, wurde jeder Patient in Doppelblind-cross over-Technik entweder mit Pirenzepin oder mit Placebo behandelt. Die Dosierung betrug 2 x 25 mg/Tag. Der Versuchsplan ermöglichte also bei jedem Patienten einen zweimal 2-wöchigen Beobachtungszeitraum. Die

- 4 -

geprüften Parameter waren Obstipation, Diarrhoe, Schafkotstuhl, wechselnde Stühle und Schleimabsonderung. Die Beurteilung der therapeutischen Wirkung erfolgte vor Beginn der Behandlung und nach jedem einzelnen Versuchsabschnitt. Jeder Patient erhielt einen weitgefächerten Score, um seine subjektiven Eindrücke festzuhalten. Diese Angaben wurden ausgewertet, wobei die generalisierten Befunde bezüglich der Einzelsymptomatik in der nachstehenden Tabelle aufgeführt sind:

| Symptom | symptomfrei oder gebessert | |
|---|---|---|
| | Gastrozepin | Placebo |
| Obstipation | 72 % | 57 % |
| Diarrhoe | 84 % | 47 % |
| Schafkotstuhl | 80 % | 51 % |
| Wechselnde Stühle | 89 % | 49 % |
| Schleimabsonderung | 83 % | 48 % |

Das oben dargelegte therapeutische Ergebnis kann dahingehend zusammengefaßt werden, daß bei allen geprüften Parametern zwischen der Placebogruppe und der Verumgruppe signifikante bis hochsignifikante Unterschieden zugunsten von Pirenzepin bestanden. Pirenzepin ist in der Indikation Colon irritabile sehr gut wirksam.

Pirenzepin löst einen krankhaften Spasmus im Darmtrakt ohne den Darm zu lähmen. Um dieses zu beweisen, bzw. um zu überprüfen, ob Pirenzepin anders als Atropin die gestörten motorischen Verhältnisse zu beeinflussen in der Lage ist und sich damit von Anticholinergika, Atropin oder Atropinderivaten unterscheidet, wurde die Dickdarmmotorik bei freiwilligen Probanden mit Neostigmin stimuliert und im Akutversuch Pirenzepin intravenös in therapeutischen Dosen verabreicht.

In einer zweiten Serie wurden die Druck- und Motorikverhältnisse bei Patienten mit Colon irritabile vor und nach einer oralen Therapie mit Pirenzepin geprüft. Die dabei erzielten Ergebnisse lassen sich wie folgt zusammenfassen: Im Vergleich zur basalen Untersuchungsperiode konnte unter Neostigminstimulation an gesunden Testpersonen eine signifikante Zunahme der Druckamplitude um 21 %, der Kontraktionsdauer um 76 % und ein Anstieg des Motilitätsindex um 82 % beobachtet werden. Pirenzepin bewirkt eine Abnahme der Druckamplitude, der Kontraktionsdauer und des Motilitätsindex bis zum Ausgangsniveau. Eine Abnahme der propulsiven Kontraktionen war dagegen nicht zu registrieren. Hieraus wurde klar der Unterschied beispielsweise zu Atropin sichtbar, wobei Atropin vor allem die propulsiven motorischen Kontraktionen zum Erliegen bringt.

Die nach Stimulierung mit Neostigmin an freiwilligen gesunden Probanden gemachten Beobachtungen konnten auch klinisch-experimentell an Patienten mit Colon irritabile bestätigt werden.

Die Herstellung von Pirenzepin und seiner Salze wird in der deutchen Patentschrift Nr. 1 795 183 (vgl. Beispiel 8) beschrieben.

Die folgenden Beispiele sollen die Herstellung einiger pharmazeutischer Anwendungsformen aufzeigen. Pirenzepin-dihydrochloridmonohydrat, im folgenden Wirkstoff genannt, läßt sich, in den obengenannten Dosen, in Tabletten, Dragées, Zäpfchen und sonstige Anwendungsformen problemlos einarbeiten.

Beispiel 1

Tabletten mit 25 mg 5,11-Dihydro-11[(4-methyl-piperazin-1-yl)acetyl]-6H-pyrido[2,3-b][1,4]benzodiazpin-6-on-dihydrochloridmonohydrat (Pirenzipin-dihydrochloridmonohydrat)

1 Tablette enthält:

| | |
|---|---|
| Wirkstoff | 25,0 mg |
| Milchzucker | 133,0 mg |
| Kartoffelstärke | 60,0 mg |
| Magnesiumstearat | 2,0 mg |
| | 220,0 mg |

Herstellungsverfahren für 1000 Tabletten:

Aus 5 g Kartoffelstärke wird mit Wasser durch Erwärmen ein 10%iger Schleim hergestellt. Die Wirksubstanz, Milchzucker und die restliche Kartoffelstärke werden miteinander vermischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

Tablettengewicht:   220 mg
Stempel:   9 mm

Beispiel 2

Dragées mit 25 mg Pirenzepin-dihydrochloridmonohydrat

Die nach Beispiel 1 hergestellten Tabletten werden nach bekannten Verfahren mit einer Hülle überzogen, die im wesentlichen aus Zucker und Talkum besteht. Die fertigen Dragées werden mit Hilfe von Bienenwachs poliert.

Dragéegewicht: 300 mg.

**Beispiel 3**

**Ampullen mit 2 mg Pirenzepin-dihydrochloridmonohydrat**

1 Ampulle enthält:

| | | |
|---|---|---|
| Wirkstoff | 2,0 | mg |
| Natriumchlorid | 8,0 | mg |
| Dest. Wasser ad | 1 | ml |

Herstellungsverfahren:

Die Wirkstubstanz und Natriumchlorid werden in destilliertem Wasser gelöst und anschließend auf das gegebene Volumen aufgefüllt. Die Lösung wird sterilfiltriert und in 1 ml-Ampullen abgefüllt.

Sterilisation: 20 Minuten bei 120°C.

**Beispiel 4**

Tabletten mit 100 mg Pirenzipin-dihydrochloridmono-hydrat

1 Tablette enthält:

| | | |
|---|---|---|
| Wirkstoff | 100,0 | mg |
| Milchzucker | 83,0 | mg |
| Kartoffelstärke | 35,0 | mg |
| Magnesiumstearat | 2,0 | mg |
| | 220,0 | mg |

Die Herstellung erfolgt wie im Beispiel 1 beschrieben.


## Beispiel 5

### Tabletten mit 10 mg Pirenzipin-dihydrochloridmonohydrat


1 Tablette enthält:

| | | |
|---|---:|---|
| Wirkstoff | 10,0 | mg |
| Milchzucker | 148,0 | mg |
| Kartoffelstärke | 60,0 | mg |
| Magnesiumstearat | 2,0 | mg |
| | 220,0 | mg |

Die Herstellung erfolgt wie im Beispiel 1 beschrieben.


## Beispiel 6

### Dragées mit 50 mg Pirenzepin-dihydrochloridmonohydrat

Tabletten, die

50,0 mg Wirkstoff
108,0 mg Milchzucker
60,0 mg Kartoffelstärke und
2,0 mg Magnesiumstearat

enthalten, werden, wie im Beispiel 2 beschrieben, mit einer Hülle überzogen und mit Bienenwachs poliert.

Dragéegewicht: 300 mg.

0155454

## Patentansprüche

1.) Verwendung von Pirenzepin oder dessen pharmakologisch verträglichen Salzen zur Behandlung des Colon irritabile.

2.) Verwendung von Pirenzepin oder dessen pharmakologisch verträglichen Salzen zur Herstellung von Arzneimitteln zur Behandlung des Colon irritabile.

3.) Arzneimittel zur Behandlung des Colon irritabile, enthaltend Pirenzepin oder dessen pharmakologisch verträgliche Salze.

4.) Verwendung von Pirenzepin-dihydrochloridmonohydrat zur Behandlung des Colon irritabile.

5.) Oral applizierbares Arzneimittel gemäß Anspruch 3, enthaltend Pirenzepin oder dessen pharmakologisch verträgliche Salze in einer Dosierung von 20 bis 50 mg pro Einzeldosis.

6.) Parenteral applizierbares Arzneimittel gemäß Anspruch 3, enthaltend Pirenzepin oder dessen pharmakologisch verträgliche Salze in einer Dosierung von 2 bis 10 mg pro Einzeldosis.

7.) Verwendung von Pirenzepin oder dessen pharmakologisch verträglichen Salzen zur Herstellung eines Arzneimittels zur Behandlung des Colon irritabile.

8.) Verfahren zur Herstellung eines Arzneimittels zur Behandlung des Colon irritabile, dadurch gekennzeichnet, daß Pirenzepin oder dessen pharmakologisch verträgliche Salze mit Träger- und/oder Hilfsstoffen zu Anwendungsformen verarbeitet werden, die bei oraler Anwendung 10 bis 100 mg des Wirkstoffes pro Dosis, bei parenteraler Anwendung 1 bis 20 mg des Wirkstoffes pro Dosis enthalten.

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 99, 1983, Seiten 52-53, Nr. 16355z, Columbus, Ohio, US; F. BALDI u.a.: "Effect of pirenzepine on colonic motility in man" & CURR. THER. RES. 1983, 33(5), 802-6 | 1 | A 61 K 31/55 // C 07 D 471/04 (C 07 D 471/04 C 07 D 243:00 C 07 D 221:00 ) |

-----

RECHERCHIERTE
SACHGEBIETE (Int. Cl.4)

A 61 K 31/00

Der vorliegende Recherchenbericht wurde für alle Patentansprüche erstellt.

| Recherchenort DEN HAAG | Abschlußdatum der Recherche 13-05-1985 | Prüfer ALFARO I. |
|---|---|---|